# EUROPÄISCHE PATENTANMELDUNG

(11) **EP 0 624 566 A1**
(43) Veröffentlichungstag der Anmeldung: **17.11.1994**
(21) Anmeldenummer: 94106956.9
(22) Anmeldetag: 04.05.1994
(51) Int. Cl.: C07C 67/04, C07C 69/54

(54) **Verfahren zur Herstellung von Isobornyl(meth)acrylat**

(30) Priorität: 13.05.1993 DE 4316004
(71) Anmelder: RÖHM GMBH, D-64293 Darmstadt (DE)
(72) Erfinder: Pfirmann, Martina, Dr., D-64347 Griesheim (DE); Knebel, Joachim, Dr., D-64293 Darmstadt (DE)

(57) **Zusammenfassung**

Die Erfindung beschreibt ein Verfahren zur Herstellung von Isobornylacrylat oder Isobornylmethacrylat durch Umsetzung von Camphen mit Acrylsäure oder Methacrylsäure in Gegenwart von Phosphormolybdän-Heteropolysäure als Katalysator.

## Beschreibung

### Gebiet der Erfindung

Die Erfindung betrifft ein Verfahren zur Herstellung von Acrylsäure- und Methacrylsäureestern durch katalytische Addition von Acrylsäure oder Methacrylsäure an Alkene. Die Erfindung betrifft insbesondere ein Verfahren zur Herstellung von Isobornylacrylat und Isobornylmethacrylat.

### Stand der Technik

An Olefine können unter saurer Katalyse bis zu einem Gleichgewichtsumsatz Carbonsäuren addiert werden. Diese Methode stellt eine Ergänzung zu den herkömmlichen Um- bzw. Veresterungsreaktionen dar, da hiermit die ansonsten schwer zugänglichen Ester der sekundären und insbesondere der tertiären Alkohole herstellbar sind. Als Katalysatoren können neben Protonensäuren auch Lewissäuren wie Bortrifluorid verwendet werden [s. Houben-Weyl, Bd. 8 (1952), S. 534). Je höher der Substitutionsgrad an der Doppelbindung (Alkylsubstituenten) und je acider die Carbonsäure ist, umso günstiger liegt dieses Gleichgewicht auf seiten des Esters.
Als Beispiele seien die Herstellung von Acetaten oder Formiaten ausgehend von Isobutylen, Isoamylen, Dicyclopentadien, Norbornen oder Camphen genannt. Die Herstellung von Isobornylacrylat aus Camphen und Acrylsäure mit H₂SO₄ als Katalysator, ist in der japanischen Patentschrift JP 79 126 293 (Ref: C.A. 92,60 517) beschrieben.

Auch saure Ionenaustauscher finden als Katalysatoren für die Umsetzung Verwendung. So ist die Herstellung von Isobornyl(meth)acrylat aus Camphen und (Meth)acrylsäure unter Verwendung eines stark sauren Ionenaustauschers in der japanischen Patentschrift JP 58,049 337 beschrieben. Jedoch zeigt es sich, daß zur Erreichung des Gleichgewichtsumsatzes lange Reaktionszeiten (> 8 Stunden) vonnöten sind. Dies bedingt eine schlechte Raum-Zeit-Ausbeute, was sich in den Herstellkosten negativ niederschlägt. Auch bereitet bei solch langen Reaktionszeiten bei höheren Temperaturen die Stabilisierung des Reaktionsgemisches (Polymerisationsvermeidung) u.U. größere Probleme.

Für die Herstellung von organischen Verbindungen durch Umsetzung von Olefinen mit Carbonsäuren, auch ungesättigten Carbonsäuren, zu den entsprechenden Estern, werden in der DE-OS 19 54 986 Heteropolysäuren des Molybdäns oder Wolframs als Katalysatoren beansprucht. So können nach der DE-A 30 34 033 auch Dihydrodicyclopentylacrylat und -methacrylat durch Umsetzung von Dicyclopentadien, einem endocyclischen Diolefin, mit Acrylsäure oder Methacrylsäure in Gegenwart einer sauren Wolframheteropolysäure-Verbindung als Katalysator hergestellt werden. Eine Übertragung der Wolframheteropolysäure-Katalyse auf die Umsetzung von Camphen mit Acrylsäure oder Methacrylsäure gelang in eigenen durchgeführten Versuchen nicht.

### Aufgabe und Lösung

Dem erwünschten größeren Einsatz der interessanten Verbindungen Isobornylacrylat und Isobornylmethacrylat, insbesondere als Comonomere zur Herstellung technisch verwendbarer Polymerisate, stehen Unzulänglichkeiten in den für deren Herstellung bekannten Herstellungsverfahren entgegen.
Lange Reaktionszeiten wie in der diskutierten Säure-Ionenaustauscher-Katalyse, Korrosionswirkungen von Mineralsäuren und von Bortrifluorid, Neutralisation dieser löslichen Katalysatoren und Waschen des Reaktionsgemisches mit Wasser und gegebenenfalls Extraktion, was die Aufarbeitung sehr kompliziert gestaltet, sind Gründe, die die technischen Umsetzungen der bekannten Verfahrensmöglichkeiten schwierig machen. Auch ist eine Destillation in Anwesenheit des Katalysators nicht möglich, da bei der destillativen Entfernung der nicht reagierten, leichterflüchtigen Säure sich der Ester wieder unter Einstellung des Gleichgewichts zersetzt.

Zur Herstellung von Isobornylacrylat und Isobornylmethacrylat aus Camphen und den beiden ungesättigten Carbonsäuren sollte ein Katalysator gefunden werden, welcher eine hohe Aktivität, was eine kurze Reaktionszeit bedeutet, aufweist, und sich auf einfache Art und Weise nach der Umsetzung abtrennen läßt und damit die Aufarbeitung durch Destillation möglich ist.

Es wurde gefunden, daß von verschiedenen geprüften Heteropolysäuren nur Phosphormolybdän-Heteropolysäure, vor allem H₃PMo₁₂O₄₀ · xH₂O die erforderliche Aktivierung, d.h. Beschleunigung der Umsetzung von Camphen mit Methacrylsäure oder Acrylsäure zu deren Isobornylestern zeigt. Mit anderen Heteropolysäuren, z.B. Vanadiumderivaten der Phosphormolybdänsäure wurde entweder keine oder nur geringe Esterbildung gefunden.

Überraschend und auch nicht vorhersehbar, daß die in der DE-A 30 34 033 beschriebene Umsetzung von Dicyclopentadien mit Acrylsäure bzw. Methacrylsäure mit dem im erfindungsgemäßen Verfahren hochwirksamen Phosphormolybdänsäure-Katalysator nicht katalysiert wird, und andererseits, wie oben schon ausgeführt, die Phosphorwolframsäure bei der Isobornyl(meth)acrylat-Bildung keine katalytische Aktivität zeigt.

Gegenstand der Erfindung ist daher ein Verfahren zur Herstellung von Isobornylacrylat oder Isobornylmethacrylat durch Umsetzung von Camphen mit Acrylsäure oder Methacrylsäure in Gegenwart eines Katalysators, dadurch gekennzeichnet, daß als Katalysator Phosphormolybdän-Heteropolysäure eingesetzt wird.

Der Heteropolysäurekatalysator wird zweckmäßig nach Beendigung der Umsetzung, zweckmäßigerweise nach Zusatz von Alkali zur Ausfällung von anteilig gelöstem Katalysator und gegebenenfalls nach Verdünnung mit einem organischen Lösemittel, vorteilhaft durch Filtration abgetrennt. Das Alkali, z.B. NaOH, KOH, NaHCO₃, NaOCH₃, wird zweckmäßigerweise als wäßrige oder auch als methanolische Lösung nach der Umsetzung und in Mengen, die der eingesetzten Heteropolysäure etwa äquivalent sind, zugesetzt. Die zurückgewonnene Heteropolysäure kann gegebenenfalls wiederholt für die Umsetzung von Camphen mit einer der ungesättigten Säuren eingesetzt werden. Gegebenenfalls kann die Umsetzung auch in einem organischen Lösemittel durchgeführt werden.

### Durchführung der Erfindung

Die für die Umsetzung zu verwendende Katalysatormenge liegt bei 0,1 bis 5 Gew.-%, vorzugsweise bei 0,1 bis 3 Gew.-% bezogen auf das Gesamtgewicht der eingesetzten Reaktionspartner. Camphen wird zweckmäßig im Molverhältnis 0,5 bis 2 Mol, insbesondere von 0,8 bis 1,2 Mol pro Mol der eingesetzten ungesättigten Säure verwendet. Die Reaktionstemperatur kann im Bereich von 40 bis 140 Grad C, vorzugsweise im Bereich von 60 bis 100 Grad C liegen. Bei Temperaturen deutlich über 100 Grad C kann eine Polymerisation, vor allem der ungesättigten Säure oder des Reaktionsproduktes eintreten, während bei Temperaturen unter 40 Grad C die Reaktion mit nur sehr geringer Geschwindigkeit abläuft. Die Umsetzungszeiten liegen bevorzugt im Bereich von 1 bis 5 Stunden.

Normalerweise wird die Umsetzung im geschlossenen System ausgeführt, in dem sich dann entsprechend der Reaktionstemperatur ein entsprechender Druck einstellt. Die Umsetzung kann aber auch bei noch höherem oder bei erniedrigtem Druck durchgeführt werden.

Das erfindungsgemäße Verfahren kann entweder chargenweise oder kontinuierlich geführt werden. Da Ausgangs- und Reaktionsprodukte polymerisieren können, werden sowohl bei der Umsetzung als auch bei der Aufarbeitung Polymerisationsinhibitoren, z.B. Hydrochin, Phenothiazin, Hydrochinonmonomethylether u.a. zugesetzt.

Die nachfolgenden Beispiele sollen das erfindungsgemäße Verfahren weiter veranschaulichen.

### BEISPIELE

### Herstellung von Isobornylmethacrylat

In einer Lösung von 204,4 g (1,5 mol) Camphen in 140 ml Methacrylsäure (1,65 mol, stabilisiert mit 60 mg Hydrochinon und 30 mg Phenothiazin) wurden 3,46 g Molybdatphosphorsäure suspendiert. Unter Lufteinleitung wurde 3 Stunden bei 80 Grad C gerührt. Nach Abkühlung auf Raumtemperatur wurde das Reaktionsgemisch mit 1,5 g konzentierter Kalilauge versetzt und vom Katalysator filtriert. Nach Destillation wurden 178 g (53 % d.Th. Kp. 74 - 80 Grad C/ca. 0,4 mbar) Isobornylmethacrylat erhalten.

### Herstellung von Isobornylacrylat

In einer Lösung von 1,36 kg (10 mol) Camphen in 0,76 l Acrylsäure (11 mol, stabilisiert mit 0,4 g Hydrochinon und 0,2 g Phenothiazin) wurden 21,6 g Molybdatphosphorsäure suspendiert. Unter Lufteinleitung wurde 3 Stunden bei 80 Grad C gerührt. Nach Abkühlung auf Raumtemperatur wurde das Reaktionsgemisch mit 9 g konzentrierter Kalilauge versetzt und vom Katalysator filtriert. Nach Destillation wurden 1050 g Isobornylacrylat (50 % d.Th.) erhalten.

## Patentansprüche

1. Verfahren zur Herstellung von Isobornylacrylat oder Isobornylmethacrylat durch Umsetzung von Camphen mit Acrylsäure oder Methacrylsäure in Gegenwart eines Katalysators,
dadurch gekennzeichnet,
daß als Katalysator Phosphormolybdän-Heteropolysäure eingesetzt wird.

2. Verfahren nach Anspruch 1, dadurch gekennzeichnet, daß die Heteropolysäure H₃PMo₁₂O₄₀ · xH₂O als Katalysator eingesetzt wird.

3. Verfahren nach den Ansprüchen 1 und 2, dadurch gekennzeichnet, daß zur weitgehenden Abtrennung des eingesetzten Katalysators nach der Umsetzung noch Alkali zugesetzt wird und die Phosphormolybdänsäure abfiltriert wird.

4. Verfahren nach den Ansprüchen 1 bis 3, dadurch gekennzeichnet, daß bei der Umsetzung und/oder der Katalysatorabtrennung noch organisches Lösemittel zugesetzt wird.
